(19) Europäisches Patentamt
European Patent Office
Office européen des brevets

(11) **EP 0 638 345 B2**

(12) **NEUE EUROPÄISCHE PATENTSCHRIFT**

(45) Veröffentlichungstag und Bekanntmachung des Hinweises auf die Entscheidung über den Einspruch:
**18.04.2001   Patentblatt 2001/16**

(45) Hinweis auf die Patenterteilung:
**12.11.1997   Patentblatt 1997/46**

(21) Anmeldenummer: **94109961.6**

(22) Anmeldetag: **28.06.1994**

(51) Int Cl.$^7$: **B01D 3/14**, C07C 33/035

(54) **Verfahren zur Durchführung von destillativen Trennungen in diskontinuierlicher Betriebsweise**

Process for performing destillative separations in a discontinuous method of operation

Procédé d'exécution de séparation par distillation pour des modes de fonctionnement discontinus

(84) Benannte Vertragsstaaten:
**BE CH DE ES FR GB IT LI NL**

(30) Priorität: **08.07.1993   DE 4322725**
**27.05.1994   DE 4418648**

(43) Veröffentlichungstag der Anmeldung:
**15.02.1995   Patentblatt 1995/07**

(73) Patentinhaber: **BASF Aktiengesellschaft**
**67063 Ludwigshafen (DE)**

(72) Erfinder:
• **Kaibel, Gerd, Dr.**
**D-68623 Lampertheim (DE)**
• **Krug, Thomas**
**D-67227 Frankenthal (DE)**
• **Ensen, Heinz-Friedrich**
**D-67227 Frankenthal (DE)**
• **Stops, Peter**
**67122 Altrip (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
**EP-A- 0 583 683**

• **R.W.ELLERBE 'Handbook of Separation Techniques...' 5. September 1989 , P.A.SCHWEITZER , NEW YORK,US * Seite 165 - Seite 166 ***
• **COMPUTERS IN INDUSTRY, Bd.13, 1989, OXFORD,GB Seiten 169 - 180 O.J.CHIOTTI ET AL. 'An Optimization Module for Batch Distillation with Intermediate Cuts'**

**Beschreibung**

Die Erfindung betrifft ein Verfahren zur Durchführung von destillativen Trennungen in diskontinuierlicher Betriebsweise, bei dem mehrere Fraktionen gemeinsam aus einem Anfangsgemisch abgetrennt und die entstehenden Kopf- und Sumpfgemische in nachfolgenden diskontinuierlichen Destillationen weiter aufgetrennt werden. Ziel der Erfindung ist es, den Energiebedarf zu senken bzw. die Destillationszeiten zu verkürzen. Die wesentliche Neuerung ist dabei die Berücksichtigung unterschiedlicher Trennsequenzen abweichend von der Normalfahrweise, bei der die einzelnen Komponenten nacheinander in der Reihenfolge ihrer Flüchtigkeit über Kopf entnommen werden.

Diskontinuierliche Trennprozesse mittels einer Destillationsblase mit aufgesetzter Destillationskolonne sind bekannt und in der Literatur in vielfältiger Art beschrieben, so beispielsweise in dem Lehrbuch "Thermische Trennverfahren" von K. Sattler, Ausgabe 1988, VCH Verlagsgesellschaft, Weinheim.

In der Fachliteratur finden sich eine Reihe von speziellen Ausgestaltungen dieses Grundverfahrens, insbesondere Fahrweisen, die Elemente der kontinuierlichen Verfahrensführung beinhalten. Dabei wird das zu trennende Ausgangsgemisch nicht über eine aufgesetzte Kolonne, sondern über eine als Abtriebskolonne geschaltete Destillationskolonne geschickt, wobei die einzelnen Fraktionen, beginnend mit der schwerstflüchtigen Fraktion, über den Sumpf der Kolonne entnommen werden.

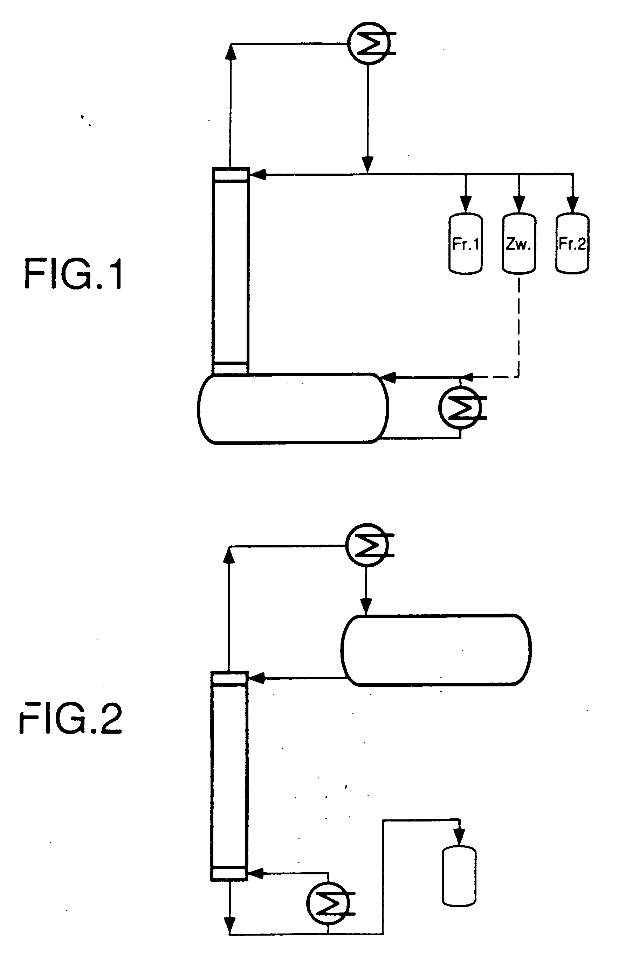

Bei der konventionellen Durchführung der diskontinuierlichen Destillation trennt man die einzelnen Fraktionen in der Reihenfolge ihrer Flüchtigkeit nacheinander über Kopf ab, wobei man zwischen den einzelnen Fraktionen Teilmengen als Zwischenläufe entnimmt, die dann dem nächsten Destillationsansatz wieder zugegeben werden (siehe Figur 1). Bei der diskontinuierlichen Fahrweise wird jedoch meist ein Mehrfaches der Energie benötigt, die bei einer kontinuierlichen Destillation erforderlich ist.

Die DE-4226905.9 beschreibt ein verbessertes Verfahren, bei dem der Energiebedarf in bestimmten Anwendungsfällen bis auf etwa das 1,1- bis 1,2-fache des Energiebedarfs einer kontinuierlichen Destillation verringert werden kann. Die Energieverringerung wird dadurch erzielt, daß die Abtrennung einzelner Fraktionen in zwei unterschiedlichen Teilschritten erfolgt, wobei im ersten Teilschritt eine Teilmenge über Kopf oder als Seitenprodukt in einer Reinheit entnommen wird, die kleiner ist als die erforderliche Endreinheit dieser Fraktion, diese Teilmenge in einem oder mehreren Behältern zwischengespeichert wird, in einem weiteren Teilschritt diese Teilmenge aus dem Behälter bzw. den Behältern entnommen, der Kolonne im mittleren Bereich zugefahren und gleichzeitig Produkt in der erforderlichen Endreinheit am Kolonnenkopf gewonnen wird.

Es zeigen sich jedoch Anwendungen, bei denen trotz Zwischenspeicherung noch eine starke Überhöhung des Energieverbrauchs gegenüber dem kontinuierlichen Vergleichsfall verbleibt.

Es stellte sich die Aufgabe, auch für diese Fälle ein diskontinuierliches Trennverfahren zu entwickeln, das hinsichtlich des Energiebedarfs einem kontinuierlichen Trennverfahren nur wenig nachsteht und welches bekannte Verfahren zur diskontinuierlichen destillativen Trennung von Mehrstoffgemischen in der Weise verbessert, daß ein niedrigerer Energieverbrauch und bezogen auf die genutzte Destillationskolonne eine hohe Produktionskapazität erreicht wird.

Es wurde gefunden, daß bei der Auftrennung eines Mehrstoffgemisches durch eine sinnvolle Wahl der Trennsequenz deutliche Energieeinsparungen bzw. verkürzte Destillationszeiten erreicht werden können. Abweichend von der Normalfahrweise, bei der die einzelnen Komponenten in der Reihenfolge ihrer Flüchtigkeit über Kopf genommen werden, werden in diesem Fall zunächst zwei oder mehr leichtsiedende Komponenten von den verbleibenden Schwersiedern abgetrennt und die entstehenden Kopf- und Sumpfgemische in nachfolgenden Destillationsschritten in der gleichen Anordnung weiter aufgetrennt.

Ein Ansatzpunkt zu weiteren Einsparungen ergibt sich bei Betrachtung der in der Fachliteratur beschriebenen Schaltungsvarianten der diskontinuierlichen Destillation. Hier erwies sich speziell die sogenannte inverse diskontinuierliche Destillation bei Einstellung spezieller Betriebszustande als nutzbar (Fig. 2). Bei ihr wird das zu trennende Ausgangsgemisch am oberen Ende der Kolonne vorgelegt, und die einzelnen Fraktionen werden nacheinander in der Reihenfolge ihrer Flüchtigkeit, beginnend mit der schwerstflüchtigen Fraktion, am unteren Kolonnenende entnommen. Die inverse Destillation wurde beispielsweise in dem Beitrag "Preferable Alternatives to Conventional Batch Distillation" von H. Abrams, M. Miladi und F. Attarwala auf dem Symposium "Distillation '87", 7.-9. September 1987 in Brighton, von R. Robinson und E. Gilliland in dem Buch "Elements of Fractional Distillation", McGraw-Hill-Verlag (1950), und von O. Chiotti et al. in Chem. Eng. Comm. 1993, Vol. 119, Seite 1 bis 21, beschrieben.

Diese Apparateanordnung wird speziell zur thermischen Schonung der zu trennenden Produkte empfohlen, da die höher siedenden Fraktionen mit minimaler Verweilzeit entnommen werden können. Daß diese Kolonnenschaltung zusätzlich besondere Vorteile hinsichtlich des Energiebedarfs bei Einstellung ganz bestimmter Betriebsbedingungen bieten kann, wurde nicht erkannt. Es wird sogar explizit als Nachteil dieser Apparateanordnung beschrieben, daß sie längere Destillationszeiten und mehr Energie benötigt. Als Grund wird angegeben, daß die Leichtsiederkomponenten ständig unter Energieaufwand nach oben destilliert werden müssen. Die Fahrweise wird daher nur für die Abtrennung temperaturempfindlicher und hochpreisiger Produkte für vertretbar gehalten.

Ferner wird dargelegt, daß die inverse diskontinuierliche Destillation dann Vorteile bieten könnte, wenn die Trennstufenzahl sehr niedrig ist und bei dem Trennproblem für den Fall einer kontinuierlichen Auftrennung in einer Kolonne mit Verstärkungs- und Abtriebsteil die benötigte Trennstufenzahl im Abtriebsteil kleiner ist als im Verstärkungsteil. Diese Aussage hielt jedoch rechnerischen Überprüfungen nicht stand. Wie weiter unten dargelegt wird, sind andere Einflußgrößen maßgebend.

Es stellte sich so die Aufgabe, die vorab aufgezeigten Nachteile zu beheben.

Diese Aufgabe wird erfindungsgemäß durch Verfahren gelöst, wie sie in den Ansprüchen definiert sind.

Hierfür kommen verschiedene apparative Anordnungen und Betriebsweisen in Betracht, die sich einzeln oder in Kombination vorteilhaft den gegebenen Trennaufgaben anpassen lassen und niedrige Energieverbrauchszahlen ermöglichen, die im Bereich einer kontinuierlichen Betriebsweise liegen.

Es werden mehrere Fraktionen gemeinsam aus dem Anfangsgemisch abgetrennt und die entstehenden Kopf- und Sumpfgemische in nachfolgenden diskontinuierlichen Destillationen weiter aufgetrennt.

Da erfindungsgemäß Abwärts- und Aufwärts Fahrweise miteinander kombiniert werden soll auf die letzteren im einzelnen eingegangen werden:

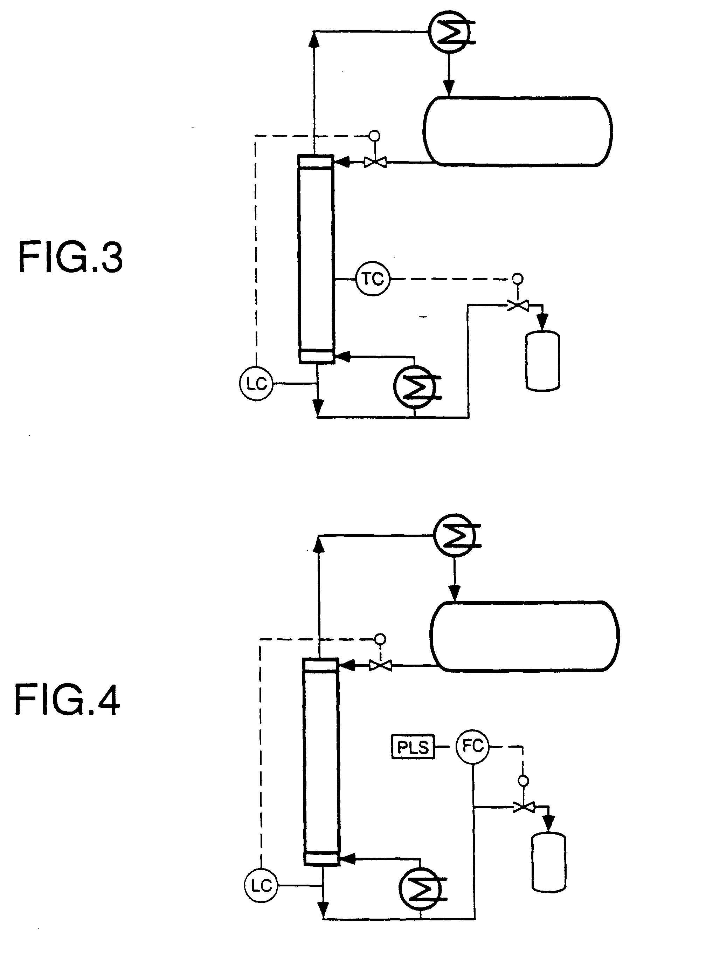

Zur Regelung einer Abtriebskolonne bieten sich mehrere Möglichkeiten an. Bei Vorhandensein eines genügend großen Siedepunktunterschieds der zu trennenden Komponenten kann eine Temperaturregelung beispielsweise gemäß Figur 3 eingesetzt werden. Bei der Abtriebsfahrweise ist die Temperaturregelung verglichen mit der herkömmlichen Fahrweise mit aufgesetzter Kolonne besser einsetzbar, da sich im Abtriebsteil deutlichere Temperaturdifferenzen einstellen als in einem Verstärkungsteil. Anstelle der Temperaturregelung kann auch eine Konzentrationsregelung durchgeführt werden.

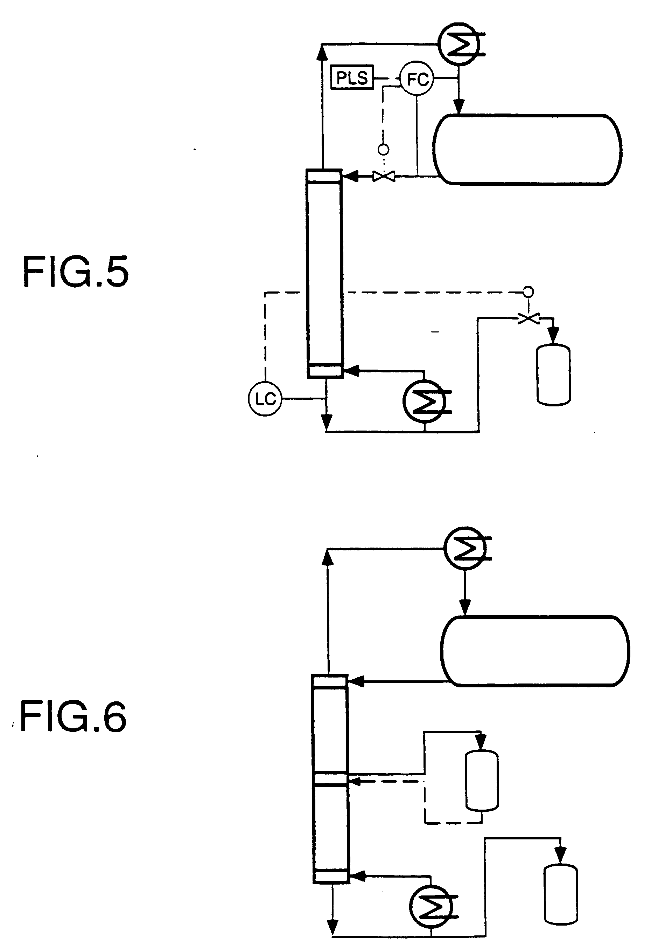

Es ist alternativ auch möglich, den zeitlichen Verlauf des Entnahmestroms vorauszuberechnen und diese Werte über ein Prozeßleitsystem oder eine Ablaufsteuerung aufzuschalten (Figur 4). Gegebenenfalls kann eine Mengendifferenzregelung eingesetzt werden (Figur 5).

Entsprechend der in der DE-4226905.9 beschriebenen Apparateanordnung einer Destillationsblase mit aufgesetzter Kolonne, bei der konventionell die gewünschten Produktfraktionen nacheinander in der Reihenfolge ihrer Flüchtigkeit über den Kopf abgetrennt werden, wobei bei den einzelnen Fraktionen eine Zwischenspeicherung von Produkt mit verminderter Reinheit vorgenommen und dieses zwischengespeicherte Produkt nachfolgend wieder in die Kolonne ejngespeist wird, kann auch bei einer gleichzeitigen Abtrennung mehrerer Fraktionen über Kopf diese Zwischenspeicherung vorgenommen werden.

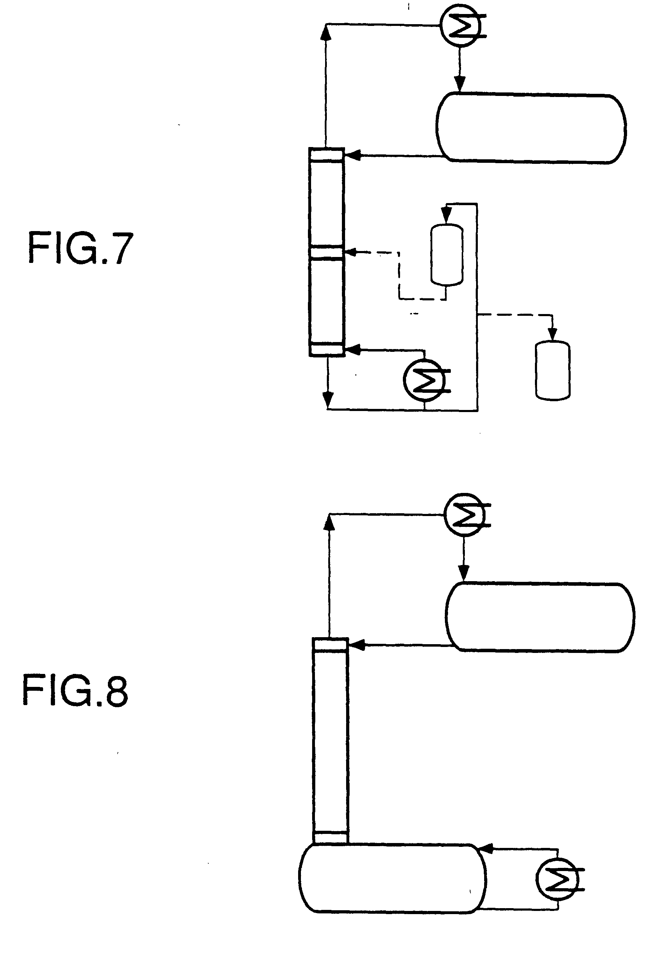

Auch bei einem Verfahren, das eine Abtriebskolonne vorsieht, kann diese Zwischenspeicherung vorteilhaft eingesetzt werden (Figur 6 und 7).

Dabei erfolgt die Abtrennung einzelner Fraktionen in zwei unterschiedlichen Teilschritten, wobei im ersten Teilschritt eine Teilmenge über Sumpf oder als Seitenprodukt in einer Reinheit entnommen wird, die kleiner ist als die erforderliche Endreinheit dieser Fraktion, diese Teilmenge in einem oder mehreren Behältern zwischengespeichert wird und anschließend in einem weiteren Teilschritt diese Teilmenge aus dem Behälter bzw. den Behältern entnommen, der Kolonne im mittleren Bereich zugefahren und gleichzeitig Produkt in der erforderlichen Endreinheit am Kolonnensumpf gewonnen wird. Bei zeitlich veränderlicher Konzentration ist es günstig, durch Einbauten im Pufferbehälter, wie z.B. Siebböden oder Füllkörper, Vermischungsvorgänge zu vermeiden. Gegebenenfalls kann die Zwischenspeicherung auf mehrere Behälter verteilt werden. Für die Rückeinspeisung kann in den meisten Fällen ein zeitlich konstanter Mengenstrom vorgesehen werden.

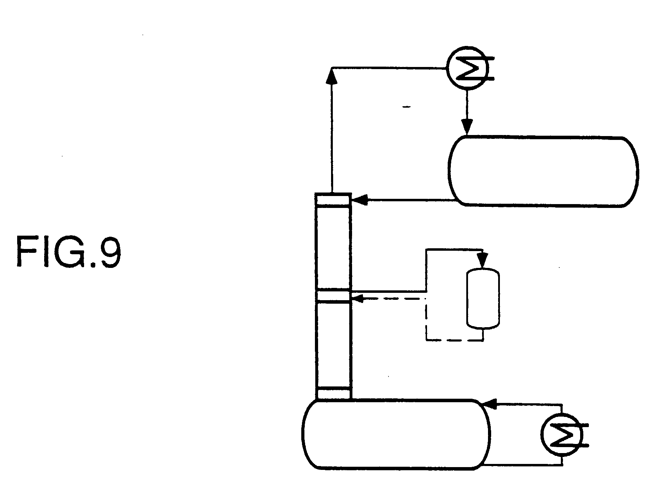

Bei Gemischen, die in drei oder mehr Einzelfraktionen zerlegt werden müssen, kann erfindungsgemäß eine Kombination der Fahrweise mit Abtriebskolonne mit der konventionellen Fahrweise mit Verstärkungskolonne nutzbringend eingesetzt werden (Figur 8). Dabei sind sowohl am oberen als auch am unteren Kolonnenende ein ausreichend groß bemessener Behälter und die notwendigen Regeleinrichtungen für Aufwärts- und Abtriebsfahrweise vorzusehen. Auch in diesem (erfindungsgemäßen) Fall ist der Einsatz eines Zwischenspeichers für Seitenprodukt mit verminderter Reinheit zweckmäßig (Figur 9).

Es ist besonders vorteilhaft, die-Entnahmemenge am oberen bzw. unteren Ende der Kolonne so einzustellen, daß sich eine zeitlich konstante Entnahmekonzentration ergibt. Diese Fahrweise führt zu den günstigsten Energieverbrauchswerten. Dabei nimmt der abgezogene Mengenstrom zeitlich ab. Erfindungsgemäß ist die Destillationskolonne sowohl am oberen als auch am unteren Ende jeweils mit einem Behälter verbunden. Das zu trennende Ausgangsgemisch wird über den mit dem oberen Ende der Destillationskolonne verbundenen Behälter zugeführt und die Leichtsiederfraktion diesem Ende entnommen, wobei in einem anschließenden Trennschritt aus dem im unteren Behälter angesammelten Gemisch aus Mittel- und Hochsiederfraktion der Mittelsieder destillativ abgetrennt und über das obere Ende der Destillationskolonne entnommen wird.

Hier wird von der Tatsache Gebrauch gemacht, daß die meisten Trennaufgaben darin bestehen, eine relativ große Menge der Mittelsiederfraktion, die in der Regel das Wertprodukt darstellt und etwa 70 bis 98% des Ausgangsgemisches entspricht, von vergleichsweise geringen Mengen von leichtersiedenden und höhersiedenden Verunreinigungen zu trennen. Es wurde gefunden, daß das erfindungsgemäße Trennverfahren bei Vorliegen der genannten Ausgangskonzentrationen unabhängig von den Siedepunktdifferenzen der zu trennenden Komponenten allgemein angewandt werden kann.

Erfindungsgemäß wird vorgesehen daß eine Destillationsvorrichtung genutzt wird, die aus einer Destillationskolonne sowie zwei Behältern besteht, wobei ein Vorlagebehälter mit dem Kolonnenkopf, der andere mit dem Kolonnensumpf verbunden ist. Das zu trennende Ausgangsgemisch wird stets in dem mit dem Kolonnenkopf verbundenen Behälter vorgelegt und in einem ersten Trennschritt dem Kopf der Destillationskolonne zugefahren. Dabei werden die mittelsiedenden und die hochsiedenden Komponenten am Sumpf der Kolonne abgetrennt und in dem Behälter am unteren Kolonnenende gesammelt. Die Leichtsiederfraktion bleibt am Kolonnenkopf zurück und wird am Ende des ersten Trennschritts ausgeschleust. In einem anschließenden zweiten Trennschritt wird aus dem am unteren Kolonnenende vorliegenden Gemisch aus Mittelsieder und Hochsieder der Mittelsieder über Kopf abdestilliert, während der Hochsieder im Kolonnensumpf zurückbleibt.

Während bei einer konventionellen Destillation mit einer aufgesetzten Kolonne das gesamte zu trennende Gemisch zunächst auf die Siedetemperatur aufgeheizt werden muß, bevor der Destillationsvorgang beginnen kann, kann bei der erfindungsgemäßen Destillation auf diesen Aufheizvorgang und den damit verbundenen Energieeinsatz verzichtet werden. Dieser Aufheizvorgang kann je nach dem zu trennenden Gemisch etwa 5 bis 20% der Destillationszeit und des Energiebedarfs betragen. Wie bereits aufgeführt, wird beim erfindungsgemäßen Verfahren am oberen Kolonnenende das Ausgangsgemisch kalt vorgelegt und zugefahren. Falls am unteren Kolonnenende von der vorhergehenden Charge kein aufgeheiztes Produkt als Mindestfüllung für den Verdampfer mehr vorhanden ist, muß zunächst lediglich eine kleine Teilmenge des Ausgangsgemisches zugefahren und bis zur Siedetemperatur aufgeheizt werden. Bezogen auf die zu trennende Gesamtmenge des Ausgangsgemisches entspricht die Mindestfüllmenge des Verdampfers nur einem kleinen Anteil, sodaß eine deutliche Verringerung des für den Aufheizvorgang benötigten Energiebetrags erreicht wird. Für die Wirtschaftlichkeit des Verfahrens ist ferner von Bedeutung, daß die Apparatebelegungszeit durch den Wegfall der Aufheizzeit verkürzt und damit die Kapazität erhöht wird.

Bei der Einführung des im ersten Trennschritt gewonnenen Hochsieders in den Vorlagebehälter kann in verschiedener Weise verfahren werden. So kann gemäß einer Ausbildung der bevorzugten Ausführungsform der kondensierte Brüden ohne Unterkühlung in den Vorlagebehälter gegeben und mit dessen Inhalt vermischt werden. Dadurch wird die enthaltene Flüssigkeitsmenge allmählich aufgeheizt. Hierdurch weist die aus dem Vorlagebehälter ablaufende Flüssigkeit, die auf den Kopf der Kolonne als Rücklauf aufgegeben wird eine abnehmende Unterkühlung auf. Dadurch stellt sich eine vorteilhafte zeitlich abnehmende Sumpfablaufmenge mit annähernd konstanter Konzentration ein. Der kondensierte Brüden kann gemäß einer anderen Ausbildung der bevorzugten Ausführungsform auch möglichst ohne Vermischung mit der kälteren und spezifisch schwereren Flüssigkeit im Vorlagebehälter überschichtet werden. Dadurch läßt sich eine stärkere zeitliche Abnahme der im Sumpf anfallenden Flüssigkeitsmenge und damit eine zeitlich konstantere Konzentration erzielen. Vorteilhafterweise wird die dem Kolonnenkopf zugeführte Flüssigkeitsmenge durch eine Regelung so eingestellt, daß sich in der ersten Destillationsphase, bei der der Mittelsieder- und Hochsiederfraktion am Sumpf abgetrennt wird, eine zeitlich konstante Sumpfkonzentration und in der zweiten Destillationsphase, bei der die Mittelsiederfraktion über Kopf abgetrennt wird, eine zeitlich konstante Kopfkonzentration einstellt.

Es ist ein besonderer Vorzug des erfindungsgemäßen Verfahrens daß die Aufheizung des Ausgangsgemisches auf Siedetemperatur nicht erforderlich ist. Falls sie dennoch gewünscht wird, kann sie ohne Auswirkung auf den Energieverbrauch mit prozeßeigener Abwärme erzielt werden, indem die Kondensatorwärme am Kolonnenkopf genutzt wird. Eine allmähliche Erwärmung des am Kolonnenkopf vorgelegten Produktes wird auch durch das heiß zulaufende und in die Vorlage eingemischte kondensierte Destillat erreicht.

Eine spezielle Ausgestaltung der Erfindung sieht vor, daß auf einen eigenen Behälter am Kolonnenkopf verzichtet wird. An dessen Stelle wird der Lagertank für das zu trennende Ausgangsgemisch benutzt. Auf diese Weise können auch konventionelle diskontinuierliche Destillationsanlagen, die nur über eine Destillationsblase am unteren Kolonnenende verfügen, mit geringem Aufwand auf die erfindungsgemäße Fahrweise umgerüstet werden.

Bei großen Kapazitäten, speziell in Fällen, bei denen bereits mehrere Destillationsanlagen zur Auftrennung in die einzelnen Fraktionen eingesetzt wurden, kann es sinnvoll sein, die erfindungsgemäße Fahrweise in zwei Apparaten durchzuführen. Dabei wird eine Apparatur für die Leichtsiederabtrennung in Abwärtsfahrweise, die andere Apparatur für die Trennung zwischen Mittel- und Hochsieder in Aufwärtsfahrweise genutzt. Bei einer solchen Fahrweise ist es besonders zweckmäßig, im Sumpf der ersten Kolonne die Mindestfüllmenge für den Verdampfer heiß zu belassen, d.h. den Behälter am Sumpf der ersten Kolonne nicht vollständig zu entleeren. Die Totzeiten für das Befüllen und Entleeren können damit vollständig vermieden werden, indem bei Erreichen der gewünschten Konzentration für den Leichtersieder am Kolonnenkopf das Kopfprodukt entleert und daran unmittelbar anschließend wieder Ausgangsgemisch zugefahren wird. Das Sumpfgemisch der ersten Kolonne weist bei der zu bevorzugenden Fahrweise eine zeitliche konstante Konzentration auf und kann daher sowohl gleichzeitig mit der Kopf-fraktion, gegebenenfalls je nach den betrieblichen Erfordernissen auch zeitlich versetzt, entnommen werden.

Ein wichtiger Vorzug des erfindungsgemäßen, Verfahrens besteht darin, daß die Mittelsiederfraktion als Wertprodukt den hohen Destillationstemperaturen nicht so lange wie bei bekannten Vergleichsverfahren ausgesetzt ist. Bei der Auftrennung temperaturempfindlicher Substanzen zeigte es sich, daß bei der Destillation deutlich weniger Rückstand gebildet wird.

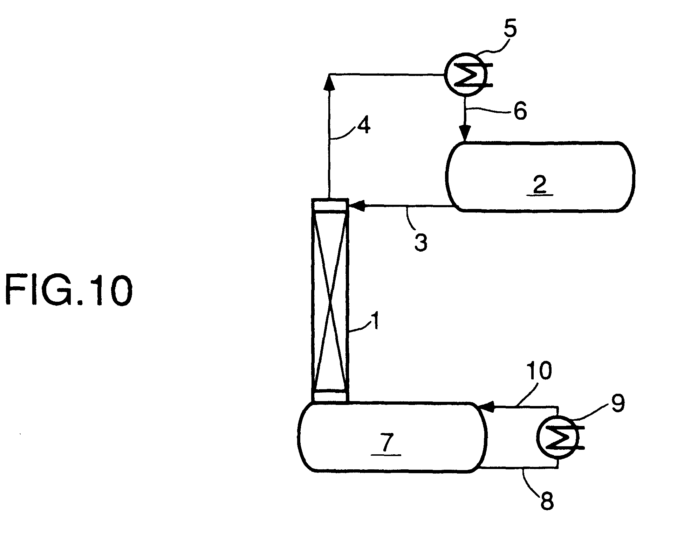

Eine schematische Darstellung der für die Durchführung des erfindungsgemäßen Verfahrens erforderlichen Apparatur ist der Figur 10 zu entnehmen. Dort ist die Destillationskolonne für die Durchführung des erfindungsgemäßen Verfahrens mit 1 bezeichnet. Diese Kolonne enthält in bekannter Weise Füllkörper oder Rektifikationsböden. Das Kopfende ist über eine Leitung 3 mit einem Behälter 2 verbunden in den das zu trennende Ausgangsgemisch eingefüllt ist. Durch eine weitere vom oberen Ende des Kopfes der Destillationskolonne 1 abgehende Leitung 4 ist ein Kondensator 5 über eine Leitung 6 mit dem Behälter 2 verbunden. Die Kolonne 1 ist auf einem als Destillationsblase dienenden Behälter 7 angeordnet. Der Inhalt dieses Behälters 7 wird über eine Leitung 8 dem Verdampfer 9 zugeführt, dort teilweise oder gänzlich verdampft und dann über die Leitung 10 wieder in den Destillationsblase 7 zurückgeführt.

Der erfindungsgemäße Betrieb dieser Anlage erfolgt in der Weise, daß zunächst das zu zerlegende Ausgangsgemisch mit den Komponenten A, B, C in den mit dem Kopf der Destillationskolonne 1 verbundenen Behälter 2 eingefüllt wird. Mit A soll im Folgenden die niedrigsiedende, mit B die mittelsiedende und mit C die hochsiedende Komponente bezeichnet werden.

Durch Öffnung eines nicht dargestellten Ventils in der Leitung 3 wird das Ausgangsgemisch dem Kopf der Destillationskolonne 1 zugeführt. Der Destillationsvorgang wird dadurch in Gang gesetzt, daß entweder noch im Behälter 7 von der vorhergehenden Destillation vorhandenes schwersiedendes oder vom Kopf der Kolonne 1 hier abfließendes oder anderweitig zugeführtes Gemisch durch den Verdampfer 9 verdampft wird und in der Destillationskolonne 1 aufsteigt, wodurch die Abtrennung der niedrigsiedenden Fraktion, die aus der Komponente A besteht, erfolgt. Diese Fraktion wird im Kondensator 5 kondensiert und über die Leitung 6 in den Behälter 2 eingeleitet. Die Temperatur ist in der Regel etwas höher als die des im Behälter 2 vorhandenen Ausgangsgemischs. Sie sammelt sich daher in einer auf diesem Gemisch liegenden Schicht. Nach Beendigung des ersten Trennschritts wird die aus dem Leichtsieder A bestehende Fraktion aus dem Behälter 2 entnommen. In einem darin anschließenden zweiten Trennschritt wird das im Behälter 7 angesammelte, aus dem im unteren Behälter 7 gesammelten Mittelsieder B und Hochsieder C bestehende Gemisch destilliert, wobei der Hochsieder C in diesem Behälter verbleibt und der Mittelsieder B in den oberen Behälter 2 abdestilliert wird. Nach Beendigung dieses zweiten Trennschritts können die Fraktionen B und C getrennt entnommen werden.

Die Auswahl der jeweils geeigneten Fahrweise in den einzelnen aufeinanderfolgenden Trennschritten - Abtriebskolonne oder Verstärkungskolonne - ist für eine gegebene Trennaufgabe im Einzelfall zu ermitteln. Zur Lösung dieser Aufgabe empfiehlt sich insbesondere eine rigorose rechnerische Simulation aller möglichen Kombinationen von Trennreihenfolgen. Anstelle der mathematischen Simulation mit Rechenprogrammen für die diskontinuierliche Destillation kann auch eine entsprechende experimentelle Untersuchung treten.

Eine vereinfachte rechnerische Abschätzung kann dadurch erfolgen, daß man für die möglichen Trennfolgen sowohl für die Aufwärtsals auch für die Abwärtsfahrweise jeweils die zur Trennung benötigten Mindestbrüdenmengen nach folgenden Beziehungen ermittelt und eine Kombination mit möglichst geringer Gesamtbrüdenmenge auswählt:

$$\text{Aufwärtsfahrweise: } G = D_{10} \frac{\alpha}{\alpha-1} \frac{p_1+p_2-1}{1-p_2} \ln\frac{1}{p_2} + D_{20} \frac{1}{\alpha-1} \frac{p_1+p_2-1}{p_1} \ln\frac{1}{1-p_1}$$

$$\text{Abwärtsfahrweise: } G = D_{10} \frac{\alpha}{\alpha-1} \frac{p_1+p_2-1}{1-p_2} \ln\frac{1}{p_2} + D_{20} \frac{1}{\alpha-1} \frac{p_1+p_2-1}{1-p_1} \ln\frac{1}{p_1}$$

Dabei bedeuten

G     zur Trennung erforderliche Mindestbrüdenmenge (mol)
$D_{10}$    Ausgangsmenge der die Leichtsiederfraktion bildenden Komponenten
$D_{20}$    Ausgangsmenge der die Schwersiederfraktion bildenden Komponenten
$p_1$     Trennausbeute der Leichtsiederkomponenten
P2    Trennausbeute der Schwersiederkomponenten
$\alpha$     relative Flüchtigkeit

Diese Beziehungen gelten für ein 2-Stoffgemisch. Für Mehrstoffgemische wird als Näherung

$$\alpha = \left( \sum_{i=1}^{i_{LS}} y_i \Big/ \sum_{i=1}^{i_{LS}} x_i \right) \Big/ \left( \sum_{j=1}^{j_{SS}} y_i \Big/ \sum_{j=1}^{j_{SS}} x_i \right)$$

gesetzt, wobei

y      die molare Brüdenkonzentration
x      die molare Flüssigkeitskonzentration
$i_{LS}$    die Zahl der Komponenten der Leichtsiederfraktion
$j_{SS}$    die Zahl der Komponenten der Schwersiederfraktion

bedeuten.

Diese weniger aufwendige Ermittlung der geeigneten Trennfolge lieferte in Beispielrechnungen brauchbare Ergebnisse. Für höhere Genauigkeitsanforderungen könnten diese Formeln abschnittsweise numerisch integriert werden.

Das Grundprinzip der vorliegenden Erfindung läßt sich auch anwenden, wenn in der Destillationskolonne und/oder den Behältern chemische Reaktionen ablaufen.

Beispiel 1 - 7 (vergleich) und 8

Für eine Untersuchung der vorstehend beschriebenen veränderten Fahrweise wurde das Gemisch n-Hexan, n-Heptan und n-Oktan verwendet. Aus einem Gemisch von 100 kg n-Hexan, 700 kg n-Heptan und 200 kg n-Oktan wurden die Reinstoffe mit einer Reinheit von 99,0 bis 99,3 Gew.-% destillativ abgetrennt (20 theoretische Trennstufen, Kopfdruck 1 bar, Druckverlust 1 mbar/Stufe). Für alle Fahrweisen wurde die Energiezufuhr auf einem konstanten Wert von 50 kW gehalten.

Insgesamt ergeben sich zur Auftrennung dieses Dreistoffgemisches bei beliebiger Verwendung der Aufwärts- und Abwärtsfahrweise acht verschiedene Fahrweisen. Dabei entspricht Fahrweise 8 dem erfindungsgemäßen Verfahren - die Fahrweisen 1 bis 7 sind als nicht erfindungsgemäße Betriebsweisen zum Vergleich aufgeführt.

| | | | Vorlage | Fahrweise | Kopfprodukt | Sumpfprodukt | Zeit [min] | Gesamtzeit [min] |
|---|---|---|---|---|---|---|---|---|
| 1 | 1. Fraktion | | Sumpf | Aufwärts | Hexan | Heptan, Oktan | 240 | |
| | 2. Fraktion | | Sumpf | Aufwärts | Heptan | Oktan | 250 | 490 |
| 2 | 1. Fraktion | | Sumpf | Aufwärts | Hexan, Heptan, | Oktan | 260 | |
| | 2. Fraktion | | Sumpf | Aufwärts | Hexan | Heptan | 220 | 480 |
| 3 | 1. Fraktion | | Sumpf | Aufwärts | Hexan | Heptan, Oktan | 240 | |
| | 2. Fraktion | | Kopf | Abwärts | Heptan | Oktan | 600 | 840 |
| 4 | 1. Fraktion | | Sumpf | Aufwärts | Hexan, Heptan, | Oktan | 260 | |
| | 2. Fraktion | | Kopf | Abwärts | Hexan | Heptan | 180 | 440 |
| 5 | 1. Fraktion | | Kopf | Abwärts | Hexan, Heptan, | Oktan | 540 | |
| | 2. Fraktion | | Kopf | Abwärts | Hexan | Heptan | 180 | 720 |
| 6 | 1. Fraktion | | Kopf | Abwärts | Hexan | Heptan, Oktan | 180 | |
| | 2. Fraktion | | Kopf | Abwärts | Heptan | Oktan | 600 | 780 |
| 7 | 1. Fraktion | | Kopf | Abwärts | Hexan, Heptan, | Oktan | 540 | |
| | 2. Fraktion | | Sumpf | Aufwärts | Hexan | Heptan | 220 | 760 |
| 8 | 1. Fraktion | | Kopf | Abwärts | Hexan | Heptan, Oktan | 180 | |
| | 2. Fraktion | | Sumpf | Aufwärts | Heptan | Oktan | 250 | 430 |

Die Ergebnisse zeigen, daß die Fahrweise 8, die günstgste ist. Im Unterschied zur klassischen diskontinuierlichen Destillation, wo die einzelnen Fraktionen in der Reihenfolge ihrer Flüchtigkeiten nacheinander über Kopf abdestilliert werden, wird hier im ersten Schritt ein Mehrstoffgemisch abgetrennt, das erst im zweiten Schritt weiter aufgearbeitet wird.

Die vorgestellten Ergebnisse basieren auf Rechnungen mit rigorosen Methoden und sind durch Versuche im Labormaßstab bestätigt.

Die beiden folgenden Beispiele lassen den mit der bevorzugten Ausführungsform der Erfindung erzielbaren technischen Fortschritt klar erkennen. Dort werden im Beispiel 9 die mit einem Verfahren nach dem Stand der Technik erzielbaren Ergebnisse bei der Zerlegung eines bestimmten Ausgangsgemischs beschrieben. Im Beispiel 10 wird dasselbe Ausgangsgemisch nach dem erfindungsgemäßen Verfahren zerlegt.

Beispiel 9 (Vergleichsbeispiel)

1. Ein Ausgangsgemisch, bestehend aus 96,6 Gew.-% Butendiol, 0,4 Gew.-% Butindiol, 1,8 Gew.-% Butandiol und 1,2 Gew.-% leichtsiedende Nebenprodukten, soll destillativ auf eine Butendiol-Reinfraktion mit einem Gehalt von über 99 Gew.-% Butendiol aufgearbeitet werden. Der Butandiolgehalt wird auf 0,5 Gew.-%, der Butindiolgehalt auf 0,3 Gew.-% begrenzt. Die Trennung erfolgt in einer Destillationsblase mit aufgesetzter Kolonne. Zu Beginn der Trennung werden 18 000 kg des Ausgangsgemisches in der Destillationsblase vorgelegt. Die Energiezufuhr beträgt stündlich 850 kg Heizdampf mit einem Druck von 17 bar. Im ersten Destillationsschritt werden in 36 h 4300 kg Leichtsiederfraktion, im zweiten Destillationsschritt in 19 h 11 800 kg Mittelsiederfraktion über Kopf abgetrennt. Als Hochsiederfraktion verbleiben 1 900 kg in der Destillationsblase. Die gesamte Destillationszeit beträgt 55 h.

Beispiel 10

2. Dasselbe Ausgangsgemisch wird wie im Beispiel 9 nach dem erfindungsgemäßen Verfahren aufgetrennt. Dabei wird das Gemisch aus einem Vorlagebehälter kalt am Kopf derselben Destillationskolonne zugefahren. Die Reinheitsanforderungen an die Mittelsiederfraktion entsprechen dem Beispiel 1. Im ersten Destillationsschritt verbleiben nach einer Destillationszeit von 26 h 1 200 kg als Leichtsiederfraktion im Vorlagebehälter am Kolonnenkopf. Im zweiten Destillationsschritt werden in 20 h 14 500 kg Mittelsiederfraktion aus der Destillationsblase am unteren Kolonnenende herausdestilliert und am oberen Kolonnenende entnommen. Als Destillationsrückstand verbleiben 2 300 kg in der Destillationsblase. Die Gesamtzeit für die Destillation beträgt 46 h.

Im Vergleich zum Beispiel 9 wird damit eine 47% höhere Kapazität erzielt, die Produktausbeute wird um 2 700 kg gesteigert und der spezifische Energieverbrauch von 4,0 auf 2,7 t Heizdampf pro t Wertprodukt gesenkt.

**Patentansprüche**

1. Verfahren zur Durchführung von destillativen Trennungen in diskontinuierlicher Betriebsweise, bei dem mehrere Fraktionen gemeinsam aus einem Anfangsgemisch abgetrennt und die entstehenden Kopf- und Sumpfgemische in nachfolgenden diskontinuierlichen Destillationen weiter aufgetrennt werden, dadurch gekennzeichnet, daß die Destillationskolonne (1) sowohl am.oberen als auch am unteren-Ende jeweils mit einem Behälter. (2;7) verbunden ist und das zu trennende Ausgangsgemisch über den mit dem oberen Ende der Destillationskolonne (1) verbundenen Behälter (2) zugeführt und die Leichtsiederfraktion diesem Ende entnommen wird und wobei in einem anschließenden Trennschritt aus dem im unteren Behälter (7) angesammelten Gemisch aus Mittel- und Hochsiederfraktion der Mittelsieder destillativ abgetrennt und über das obere Ende der Destillationskolonne (1) entnommen wird, wobei die Mittelsiederfraktion mindestens 70 Gew.-% des zu trennenden Ausgangsgemisches ausmacht.

2. Verfahren nach Anspruch 1, dadurch gekennzeichnet, daß das zu Beginn der Trennung kalt vorgelegte Ausgangsgemisch in einem Wärmetauscher mit der Kondensationswärme des Brüdens am Destillationskopf der Destillationskolonne aufgeheizt wird.

3. Verfahren nach Anspruch 1 oder 2, dadurch gekennzeichnet, daß als Vorlagebehälter für das zu trennende Ausgangsgemisch ein Lagertank dient, der mit dem oberen Ende der Destillationskolonne verbunden wird.

4. Verfahren nach einem der Ansprüche 1 bis 3, dadurch gekennzeichnet, daß zwei Destillationsapparaturen verwendet werden, wobei in der ersten Apparatur das zu trennende Ausgangsgemisch dem Kolonnenkopf zugeführt, das Gemisch aus Mittelsieder und Hochsieder am unteren Ende entnommen, dem Sumpf der zweiten Destillationsapparatur zugeführt und die Mittelsiederfraktion dort über Kopf abgetrennt wird.

5. Verfahren nach Anspruch 4, dadurch gekennzeichnet, daß das Sumpfprodukt der ersten Kolonne nicht vollständig entleert, sondern die für das Betreiben des Verdampfers erforderliche Mindestfüllmenge im Sumpfbehälter-heiß belassen und als Startfüllung der nächsten Charge verwendet wird.

**6.** Verfahren nach einem der Ansprüche 1 bis 5, dadurch gekennzeichnet, daß das zu trennende Ausgangsgemisch zunächst kalt in dem mit dem Kopf der Kolonne verbundenen Vorlagebehälter vorgelegt und in einem zeitlich konstanten Mengenstrom dem Kopf der Kolonne zugeführt wird, der kondensierte Brüden ohne Unterkühlung in den Vorlagebehälter gegeben, mit dessen Inhalt vermischt und die enthaltene Flüssigkeitsmenge dadurch allmählich aufgeheizt wird, wodurch die aus dem Vorlagebehälter ablaufende Flüssigkeit, die am Kolonnenkopf als Rücklauf aufgegeben wird, eine abnehmende Unterkühlung aufweist und sich dadurch eine zeitlich abnehmende Sumpfablaufmenge mit annähernd konstanter Konzentration einstellt.

**7.** Verfahren nach Anspruch 6, dadurch gekennzeichnet, daß der kondensierte Brüden möglichst ohne Vermischung mit der kälteren und damit spezifisch schwereren Flüssigkeit im Vorlagebehälter überschichtet wird.

**8.** Verfahren nach einer der Ansprüche 1 bis 5, dadurch gekennzeichnet, daß die dem Kolonnenkopf zugeführte Flüssigkeitsmenge durch eine Regelung so eingestellt wird, daß sich in der ersten Destillationsphase,-bei der di Mittelsieder- und Hochsiederfraktion am Sumpf abgetrennt wird, eine zeitliche konstante Sumpfkonzentration un in der zweiten Destillationsphase, bei der die Mittelsiederfraktion über Kopf abgetrennt wird, eine zeitlich kenstani Kopfkonzentration einstellt.

**9.** Verfahren nach einem der Ansprüche 1 bis 8, dadurch gekennzeichnet, daß das zu trennende Gemisch aus 0,1 bis 4 Gew.-% Butandiol, 0,1 bis 4 Gew.-% Butindiol, 0,1 bis 4 Gew.-% hochsiedendem Rückstand und 88 bis 99,7 Gew.-% Butendiol enthält.

**Claims**

**1.** A process for carrying out distillative separations by a batchwise procedure, in which a plurality of fractions are separated off together from a starting mixture and the resulting top and bottom mixtures are further separated in downstream batchwise distillations, wherein the distillation column (1) is connected, at both the top and the bottom end, to a container (2; 7) and the starting mixture to be separated is fed via the container (2) connected to the top end of the distillation column (1), and the low-boiling fraction is removed from this end, and, in a subsequent separation step, the medium boiler is separated off by distillation from the mixture collected in the bottom container (7) and consisting of medium-boiling and high-boiling fraction and is removed via the top end of the distillation column (1), the medium-boiling fracfion accounting for at least 70 % by weight of the starting mixture to be separated.

**2.** A process as claimed in claim 1, wherein the starting mixture initially taken at room temperature at the beginning of the separation is heated in a heat exchanger with the heat of condensation of the vapor at the top of the distillation column.

**3.** A process as claimed in claim 1 or 2, wherein a storage tank which is connected to the top end of the distillation column serves as a receiver for the starting mixture to be separated.

**4.** A process as claimed in any of claims 1 to 3, wherein two distillation apparatuses are used, in the first apparatus the mixture to be separated being fed to the top of the column, the mixture consisting of medium boiler and high boiler being removed at the bottom end and fed to the bottom of the second distillation apparatus, and the medium-boiling fraction being separated off there via the top.

**5.** A process as claimed in claim 4, wherein the bottom product of the first column is not completely emptied but the minimum content required for operation of the evaporator is left at elevated temperatures in the bottom container and is used as an initial content for the next batch.

**6.** A process as claimed in any of claims 1 to 5, wherein the starting mixture to be separated is initially taken at room temperature in the receiver connected to the top of the column and is fed to the top of the column at a flow rate which is constant as a function of time, the condensed vapors are introduced into the receiver without cooling and are mixed with the content thereof, and the amount of liquid contained is gradually heated up, with the result that the liquid which flows out of the receiver and is introduced as reflux at the top of the column exhibits decreasing cooling and a bottom outflow which decreases as a function of time and has a virtually constant concentration is thus obtained.

7.  A process as claimed in claim 6, wherein the condensed vapors are collected as a top layer in the receiver, as far as possible without mixing with the colder liquid which therefore has a higher specific gravity.

8.  A process as claimed in any of claims 1 to 5, wherein the amount of liquid fed to the top of the column is adjusted by regulation so that a bottom concentration which is constant as a function of time is obtained in the first distillation phase, in which the medium-boiling and high-boiling fraction is separated off at the bottom, and a top concentration which is constant as a function of time is obtained in the second distillation phase, in which the medium-boiling fraction is separated off via the top.

9.  A process as claimed in any of claims 1 to 8, wherein the mixture to be separated consists of from 0.1 to 4 % by weight of butanediol, from 0.1 to 4 % by weight of butynediol, from 0.1 to 4 % by weight of high-boiling residue and from 88 to 99.7 % by weight of butenediol.

**Revendications**

1.  Procédé d'exécution de séparations par distillation dans un mode de fonctionnement discontinu, procédé dans lequel plusieurs fractions sont séparées conjointement à partir d'un mélange initial et les mélanges de tête et de queue se constituant étant l'objet d'une séparation supplémentaire opérée lors de distillations subséquentes faites de façon discontinue, caractérisé par le fait que la colonne de distillation (1) est reliée, tant à l'extrémité supérieure qu'également à l'extrémité inférieure, à un récipient (2; 7) et le mélange initial à séparer étant amené, à travers le récipient (2) relié à l'extrémité supérieure de la colonne de distillation (1), et la fraction aisément évaporable étant prélevée de cette extrémité, étant séparée par voie de distillation lors d'une étape de séparation subséquente, à partir du mélange, accumulé dans le récipient inférieur (7) et constitué de la fraction à point de fusion médian et à point de fusion élevé, pour la séparer des éléments à point d'ébullition moyen et prélevée par l'extrémité supérieure de la colonne de distillation (1), la fraction à point d'ébullition moyen constituant au moins 70 % en poids du mélange initial à séparer.

2.  Procédé selon la revendication 1, caractérisé par le fait que le mélange initial se présentant à froid au début de la séparation est chauffé dans un échangeur de chaleur, en utilisant la chaleur de condensation des buées se trouvant sur la tête de distillation de la colonne de distillation.

3.  Procédé selon la revendication 1 ou 2, caractérisé par le fait que l'on utilise, comme récipient collecteur pour le mélange initial à séparer, un réservoir de stockage relié à l'extrémité supérieure de la colonne de distillation.

4.  Procédé selon l'une des revendications 1 à 3, caractérisé par le fait qu'on utilise deux appareils de distillation, le mélange initial à séparer étant amené à la tête de colonne dans le premier appareil, le mélange constitué du produit à point d'ébullition moyen et à point d'ébullition élevé étant prélevé à l'extrémité inférieure, amené à la queue du deuxième appareil de distillation et la fraction à point d'ébullition moyen étant soumise à cet endroit à une séparation en partie haute.

5.  Procédé selon la revendication 4, caractérisé par le fait que le produit de queue de la première colonne n'est pas complètement vidangé mais que, au contraire, la quantité de remplissage minimale nécessaire pour le fonctionnement de l'évaporateur étant laissée, chaude, dans le récipient de queue et utilisée comme charge de démarrage pour la charge suivante.

6.  Procédé selon l'une des revendications 1 à 5, caractérisé par le fait que le mélange initial à séparer est d'abord placé à froid dans le récipient collecteur relié à la tête de la colonne et est amené à la tête de la colonne, en un débit-masse temporellement constant, les buées condensées étant placées sans surrefroidissement dans le récipient collecteur, étant mélangées à son contenu et la quantité de liquide contenue étant chauffée progressivement, faisant que le liquide s'écoulant du récipient collecteur, qui a été amené à titre de retour à la tête de colonne, présente un surrefroidissement allant en diminuant et que, de ce fait, s'établit un débit d'évacuation de queue, allant en diminuant en fonction du temps et ayant une concentration à peu près constante.

7.  Procédé selon la revendication 6, caractérisé par le fait que les buées condensées sont recouvertes, autant que possible sans mélange, par le liquide, plus froid et ainsi ayant une masse volumique plus grande, dans le récipient collecteur.

8. Procédé selon l'une des revendications 1 à 5, caractérisé par le fait que la quantité de liquide amenée à la tête de colonne est réglée au moyen d'une régulation, de manière que, dans la première phase de distillation, lors de laquelle les fractions à point d'ébullition moyen et à point d'ébullition élevé sont séparées en queue, il s'établisse une concentration temporellement constante en queue et que, dans la deuxième phase de la distillation, pour laquelle on effectue à la tête une séparation de la fraction à point d'ébullition moyen, s'établisse une concentration en tête temporellement constante.

9. Procédé selon l'une des revendications 1 à 8, caractérisé par le fait que le mélange à séparer est constitué de 0,1 à 4 % en poids de butandiol, 0,1 à 4 % en poids de butindiol, 0,1 à 4 % en poids de résidu à point d'ébullition élevé et de 88 à 99,7 % en poids de butendiol.

FIG.1

Fr.1  Zw.  Fr.2

FIG.2

FIG.3

FIG.4

FIG.5

FIG.6

# FIG.7

# FIG.8

FIG.9

FIG.10